# EUROPÄISCHE PATENTSCHRIFT

(11) **EP 2 911 592 B1**
(45) Veröffentlichungstag und Bekanntmachung des Hinweises auf die Patenterteilung: **20.12.2017**
(21) Anmeldenummer: 13783889.2
(22) Anmeldetag: 24.10.2013
(51) Int. Cl.: A61B 17/02, A61F 2/24

(54) **STABILISATOR FÜR OPERATIONEN AM SCHLAGENDEN HERZEN**
STABILIZER FOR OPERATIONS ON THE BEATING HEART
STABILISATEUR POUR OPÉRATIONS À COEUR BATTANT

(30) Priorität: 29.10.2012 DE 102012219752
(43) Veröffentlichungstag der Anmeldung: 02.09.2015
(73) Patentinhaber: Aesculap AG, 78532 Tuttlingen (DE)
(72) Erfinder: MORALES, Pedro, 78532 Tuttlingen (DE); SCHABERT, Stefanie, 78554 Aldingen (DE); CREMER, Jochen, 24226 Heikendorf (DE)
(74) Vertreter: Koller, Tobias Kilian
(86) Internationale Anmeldenummer: PCT/EP2013/072244
(87) Internationale Veröffentlichungsnummer: WO 2014/067841

(56) Entgegenhaltungen:
- WO-A1-98/27869
- DE-U1- 20 020 598
- US-A- 6 152 874
- US-A1- 2003 060 685
- US-A1- 2004 171 917

## Beschreibung

Die vorliegende Erfindung betrifft einen Stabilisator für Operationen am schlagenden Herzen und insbesondere einen Stabilisator zur temporären und partiellen Ruhigstellung des Herzens bei einer Anastomose an einem Koronargefäß.

Im Stand der Technik sind verschiedene Vorrichtungen bekannt, um einen Bereich eines Herzens insbesondere bei einer Anastomose am schlagenden Herz temporär zu Stabilisieren. So ist beispielsweise in der Veröffentlichung US 2004/0143168 A1 ein Stabilisator gezeigt, bei dem ein Gewebekontaktabschnitt gelenkig an einem Befestigungsschaft angebracht ist. Der Gewebekontaktabschnitt verfügt über zwei Auflageelemente, welche sich parallel zueinander von einem Basisabschnitt aus erstrecken. Die beiden Auflageelemente sind an ihren freien Enden und den Enden, mit denen sie an dem Basisabschnitt angebracht sind, skispitzenartig aufgebogen, um eine Beschädigung des stabilisierten Gewebes insbesondere im Endbereich der Auflageelemente zu verhindern.

Auch in den Veröffentlichungen US 2004/0171917 A1 und US 2002/0099268 A1 ist ein Stabilisator gezeigt, der zwei skiartige Auflageelemente aufweist, die an einem gemeinsamen Basisabschnitt vorgesehen sind, welcher an dem Befestigungsschaft montiert ist. In diesem Fall ist der Befestigungsschaft durch einen beweglichen und verformbaren Arm ersetzt. Auch hier ist das Gewebekontaktbauteil gelenkig an dem Arm angebracht.

Die Stabilisationsfunktion und damit die Haltefunktion der Oberfläche des Herzens, an dem die Anastomose oder eine andere Operation vorgenommen werden soll, wird dadurch erreicht, dass das Gewebekontaktbauteil und insbesondere die Auflageabschnitte gegen die Herzoberfläche gedrückt wird. Auf diese Weise wird das Operationsgebiet in das Herz hinein gedrückt und befindet sich dann am Boden einer wannenförmigen Vertiefung in der Herzoberfläche. Da bei einer Anastomose aber auch bei den meisten anderen Operationen am Herzen • insbesondere am schlagenden Herzen • Blut aus den geöffneten Gefäßen austritt, sammelt sich dieses Blut in der wannenförmigen Vertiefung, welche durch den jeweiligen Stabilisator ausgebildet wird. Damit der Operateur eine ausreichende Sicht auf das Operationsgebiet hat, muss die Operation gelegentlich kurz unterbrochen werden, damit das Blut abgesaugt werden kann. Neben Blut können sich auch andere Flüssigkeiten • insbesondere Körperflüssigkeiten • in dem Operationsgebiet sammeln. Wenn im Folgenden von abzusaugendem oder abgesaugtem Blut die Rede ist, schließt dies andere Flüssigkeiten und Körperflüssigkeiten stets mit ein.

Es ist daher das Ziel der vorliegenden Erfindung, einen Stabilisator insbesondere für Operationen am Herzen, bevorzugt am schlagenden Herzen, bereit zu stellen, bei dem eine Unterbrechung der Operation zum Absaugen von sich ansammelndem Blut nicht erforderlich ist.

Die Aufgabe der vorliegenden Erfindung wird gelöst durch einen Stabilisator gemäß Anspruch 1. Vorteilhafte Ausgestaltungen und Weiterentwicklungen des erfindungsgemäßen Stabilisators sind Gegenstand der abhängigen Ansprüche.

Der erfindungsgemäße Stabilisator, der für Operationen am Herzen und insbesondere am schlagenden Herzen geeignet ist, weist einen Befestigungsschaft und ein Gewebekontaktbauteil auf. Das Gewebekontaktbauteil verfügt über zwei im Wesentlichen parallele, längliche Auflageelemente, welche an einem Basisabschnitt vorgesehen sind. Damit die Sichtbehinderung des Operateurs durch den Befestigungsschaft möglichst gering ist, erstrecken sich die beiden Auflageelemente zunächst in eine Richtung weg von dem Basisabschnitt und sind dann um im Wesentlichen 180° umgebogen, um sich in die Gegenrichtung zu erstrecken. Die Auflageelemente sind daran angepasst, auf zwei gegenüberliegenden Seiten eines Blutgefäßes angeordnet zu sein, sodass zumindest ein Abschnitt des Blutgefäßes zwischen den beiden Auflageelementen hindurch verläuft. Indem die Auflageelemente in die Oberfläche des Herzens eingedrückt werden, kann so eine laterale Spannung auf das Operationsgebiet zwischen den beiden Auflageelementen ausgeübt werden. Auf diese Weise können Schnitte im Operationsgebiet leichter vorgenommen werden. Außerdem ist der Basisabschnitt des Gewebekontaktbauteils an einem Ende des Befestigungsschafts angebracht. Mindestens eines der beiden Auflageelemente weist einen Schlitz auf, der sich in Längsrichtung des Auflageelements zumindest über einen Abschnitt desselben erstreckt. Vorzugsweise erstreckt sich der Schnitt bis in die Nähe des freien Endes des jeweiligen Auflageelements, wobei die beiden Teile des Auflageelements an dem freien Ende verbunden sind. Weiter vorzugsweise ist der Aufbau am entgegen gesetzten Ende der Auflageelemente ähnlich. Der Befestigungsschaft weist darüber hinaus einen axial verlaufenden Kanal auf, welcher sich zumindest über einen Abschnitt des Befestigungsschafts erstreckt und im Bereich desjenigen Endes des Befestigungsschafts, an welchem der Basisabschnitt angebracht ist, mit mindestens einem Verbindungskanal in Verbindung steht. Dieser Verbindungskanal verbindet den axial verlaufenden Kanal mit der Umgebung des Befestigungsschafts. Das axiale Ende des Befestigungsschafts ist vorzugsweise verschlossen. Der mindestens eine Verbindungskanal kann radial in dem Befestigungsschaft angeordnet sein, er kann aber auch schräg angeordnet sein.

Mit einem solchen Stabilisator kann ein Bereich eines Herzens stabilisiert werden. Wenn die Öffnungen des mindestens einen Verbindungskanals an der Außenfläche des Befestigungsschafts nah zu der Auflagefläche der Auflageelemente vorgesehen sind, kann über diese Öffnungen das Blut aus dem Operationsgebiet abgesaugt werden, indem eine Saugvorrichtung bzw. eine Unterdruckvorrichtung an dem anderen Ende des axialen Kanals in dem Befestigungsschafts vorgesehen wird.

Gemäß einer vorteilhaften Ausbildung des erfindungsgemäßen Stabilisators verfügt dieser über eine Absaugeinrichtung, die lösbar an dem Befestigungsschaft anbringbar ist. Insbesondere kann diese Absaugeinrichtung auf den Befestigungsschaft von dessen freiem Ende her aufgeschoben werden. Die Absaugeinrichtung weist ein Verbindungsbauteil, mit dessen Hilfe die Absaugeinrichtung lösbar an dem Stabilisator befestigbar ist und welches wenigstens einen Verbindungshohlraum aufweist, und mindestens ein Absaugbauteil auf, welches einen axial verlaufenden Kanal aufweist, der mit dem wenigstens einen Verbindungshohlraum verbunden ist. Das Absaugbauteil ist daran angepasst, zumindest teilweise in einem Schlitz eines Auflageelements angeordnet zu sein, wenn die Absaugeinrichtung an dem Stabilisator angebracht ist. Wenn die Absaugeinrichtung auf den Befestigungsschaft aufschiebbar bzw. aufsteckbar ist, weist das Verbindungsbauteil eine Durchgangsbohrung auf, die dem Außenquerschnitt des Befestigungsschafts entspricht.

Gemäß einer weiteren vorteilhaften Ausbildung des erfindungsgemäßen Stabilisators weist wenigstens ein Auflageelement mindestens eine Nut auf, welche sich in der Auflagefläche des Auflageelements im Wesentlichen quer zu dessen Längsrichtung erstreckt. Diese Nut ermöglicht es dem Blut, welches sich in dem Operationsgebiet gesammelt hat, unter dem Auflageelement hindurch zu der Absaugeinrichtung hin zu strömen. Auf diese Weise kann nicht nur das Blut abgesaugt werden, welches über die Auflageelemente strömt. Das Ergebnis der Absaugung wird hierdurch verbessert.

Gemäß einer anderen vorteilhaften Ausbildung des erfindungsgemäßen Stabilisators weist das mindestens eine Absaugbauteil der Absaugeinrichtung mindestens eine radiale Öffnung auf, die mit einer Nut in dem Auflageelement in Verbindung steht, wenn die Absaugeinrichtung lösbar an dem Stabilisator angebracht ist und das Absaugbauteil teilweise in dem Schlitz in dem Auflageelement angeordnet ist. Eine solche radiale Nut ist besonders Vorteilhaft, da die Flüssigkeit so über einen längeren Bereich in Längsrichtung der Anlageelemente angesaugt werden kann und nicht nur an der axialen Öffnung des Absaugbauteils. Wenn mindestens eine radiale Öffnung in dem Absaugbauteil vorgesehen ist, kann das axiale Ende des Absaugbauteils auch verschlossen sein. Weiterhin ist es besonders vorteilhaft, wenn die radialen Öffnungen mit den Nuten in den Auflageflächen der Auflageelemente in Verbindung stehen, da so das Blut auch direkt aus dem Operationsgebiet zwischen den beiden Anlageelementen zu dem oder den Absaugbauteil(en) angesaugt werden kann. Besonders vorteilhaft ist es, wenn mehrere radiale Öffnungen in Längsrichtung verteilt in dem Absaugbauteil angeordnet sind und entsprechend auch mehrere lateral verlaufende Nuten in der Auflagefläche der Auflageelemente ausgebildet sind. Zudem kann an dem Absaugbauteil auch eine axiale Öffnung vorgesehen sein, die verhindert, dass das Gewebe, auf dem das Absaugbauteil aufliegt, durch die radialen Öffnungen an das Absaugbauteil angesaugt und dadurch möglicherweise beschädigt wird.

Gemäß einer besonders vorteilhaften Ausbildung des erfindungsgemäßen Stabilisators weisen beide Auflageelemente je einen längs verlaufenden Schlitz auf und die Absaugeinrichtung weist zwei Absaugbauteile auf, welche mit dem Verbindungsbauteil verbunden sind und daran angepasst sind, zumindest teilweise in dem entsprechenden Schlitz des Auflageelements angeordnet zu sein, wenn die Absaugeinrichtung an dem Stabilisator angebracht ist. Auf diese Weise wird ein Stabilisator mit einer Absaugeinrichtung geschaffen, welche beiderseits des Operationsgebiets Blut absaugen kann.

Gemäß einer anderen vorteilhaften Ausbildung des erfindungsgemäßen Stabilisators besteht das Absaugbauteil aus einem gebogenen Rohr. Das freie Ende des Rohres weist vorzugsweise keine scharfen Kanten auf und der Außendurchmesser des Rohres ist in etwa genau so groß wie die Dicke der Anlageelemente. Auf diese Weise wird ein Absaugbauteil ausgebildet, das besonders einfach herzustellen ist und besonders gut in den Anlagebauteilen aufgenommen werden kann und durch diese geschützt wird.

Gemäß noch einer besonders vorteilhaften Ausbildung des erfindungsgemäßen Stabilisators ist die Auflagefläche der Auflageelemente in Längsrichtung und/oder in Querrichtung der Auflageelemente so ausgebildet, dass die Ränder der Auflagefläche abgerundet bzw. skispitzenartig aufgebogen sind. Auf diese Weise kann verhindert werden, dass im Randbereich der Auflageelemente ein besonders hoher Druck auf die Oberfläche des Herzens ausgeübt wird, was zu Nekrosen führen könnte. Scharfe Kanten könnten aber auch Schnittverletzungen am Herzen hervorrufen. Mit dem vorstehend beschriebenen Aufbau der Auflageelemente bzw. deren Unterseite sind Beschädigungen des Herzgewebes weitestgehend verhindert. Der Begriff skispitzenartig aufgebogen bedeutet hierbei nicht unbedingt, dass das Bauteil nach seiner Herstellung gebogen werde muss, das jeweilige Bauteil kann auch gleich mit einer Biegung ausgebildet sein. Vielmehr bezieht sich der Begriff skispitzenartig aufgebogen auf eine seitliche Betrachtung eines Skis, bei der man die sich vom Untergrund sanft abhebende Spitze des Skis erkennt, wobei kein Knick oder keine Kante zwischen Skispitze und Auflagefläche des Skis ausgebildet ist.

Gemäß einer besonders vorteilhaften Ausbildung des erfindungsgemäßen Stabilisators verfügt der Befestigungsschaft an seinem freien Ende über einen Anschluss, an dem eine Unterdruckvorrichtung anbringbar ist, wobei der Anschluss vorzugsweise ein LUER-Anschluss ist. Das Blut wird also durch den Befestigungsschaft hindurch abgesaugt, wenn an dem freien Ende des Befestigungsschafts ein geringerer Druck angelegt wird, wir er im Bereich der Verbindungsöffnung und/oder der Absaugeinrichtung vorherrscht.

Gemäß noch einer besonders vorteilhaften Ausbildung des erfindungsgemäßen Stabilisators weist der Befestigungsschaft im Bereich des Endes, an dem der Basisabschnitt angebracht ist, mindestens eine umlaufend Nuten auf, die in der Nähe des mindestens einen Verbindungskanals angeordnet ist und daran angepasst ist, ein Dichtungselement aufzunehmen. Vorzugsweise weist das Verbindungsbauteil zwei umlaufende Nuten auf, die an seiner axialen Durchgangsöffnung derart ausgebildet sind, dass eine von diesen umlaufenden Nuten einer umlaufenden Nut des Befestigungsschafts gegenüber steht, wenn die Absaugeinrichtung an dem Stabilisator abgebracht ist, wobei die beiden Nuten daran angepasst sind, ein Dichtungsbauteil, vorzugsweise einen 0-Ring, aufzunehmen. Die Nuten des Befestigungsschafts oder des Verbindungsbauteils sind daran angepasst, dass die Dichtungsbauteile darin verbleiben, wenn die Verbindung zwischen Befestigungsschaft und Absaugeinrichtung gelöst wird. Das Verbindungsbauteil weist weiter vorzugsweise eine weitere umlaufende Nut zwischen den beiden anderen umlaufenden Nuten auf, welche den Verbindungshohlraum zumindest teilweise bildet. Auf diese Weise kann ein Stabilisator geschaffen werden, bei dem die Dichtungsbauteile • vorzugsweise 0-Ringe • zwischen Befestigungsschaft und Absaugeinrichtung gleichzeitig als Positionierhilfe und als Befestigungsmittel dienen. Zudem wird ein Verbindungshohlraum geschaffen, der ggf. beide Absaugrohre und beide Verbindungskanäle miteinander verbindet und somit weniger verstopfungsgefährdet ist. Es können auch zwei umlaufende Nuten beiderseits des Verbindungskanals in dem Befestigungsschaft vorgesehen sein.

Ein weiterer Vorteil der vorliegenden Erfindung liegt darin, dass das Herzgewebe durch den Unterdruck in

Weitere Vorteile und Merkmale der Erfindung sind dem Fachmann aus den beigefügten Figuren und der detaillierten Beschreibung der Ausführungsbeispiele ersichtlich.
- Fig. 1: zeigt eine isometrische Ansicht eines Stabilisators gemäß einem ersten Ausführungsbeispiels;
- Fig. 2A: zeigt eine isometrische Ansicht einer Absaugeinrichtung für den Stabilisator der Fig. 1;
- Fig. 2B: zeigt eine weitere isometrische Ansicht einer Absaugeinrichtung für den Stabilisator der Fig. 1;
- Fig. 3: zeigt eine isometrische Ansicht eines Stabilisators mit einer Absaugeinrichtung gemäß dem ersten Ausführungsbeispiel;
- Fig. 4: zeigt einen Ausschnitt eines Stabilisators mit einer Absaugeinrichtung gemäß der Fig. 3 von unten;
- Fig. 5: zeigt einen Ausschnitt eines Stabilisators mit einer Absaugeinrichtung gemäß der Fig. 3 mit teilweise freigeschnittener Absaugeinrichtung;
- Fig. 6A: zeigt eine isometrische Ansicht eines Stabilisators ohne Absaugeinrichtung gemäß dem ersten Ausführungsbeispiel in einem Herstellungszustand;
- Fig.6B: zeigt eine isometrische Ansicht eines Stabilisators ohne Absaugeinrichtung gemäß dem ersten Ausführungsbeispiel in einem Verwendungszustand;
- Fig. 7A: zeigt eine schematische Seitenansicht eines Stabilisators ohne Absaugeinrichtung gemäß einem zweiten Ausführungsbeispiel;
- Fig. 7B: zeigt eine schematische Ansicht eines Stabilisators ohne Absaugeinrichtung gemäß dem zweiten Ausführungsbeispiel in Richtung der Anlageelemente;
- Fig. 8A: zeigt eine schematische Seitenansicht eines Stabilisators ohne Absaugeinrichtung gemäß einem dritten Ausführungsbeispiel; und
- Fig. 8B: zeigt eine schematische Ansicht eines Stabilisators ohne Absaugeinrichtung gemäß dem dritten Ausführungsbeispiel in Richtung der Anlageelemente.

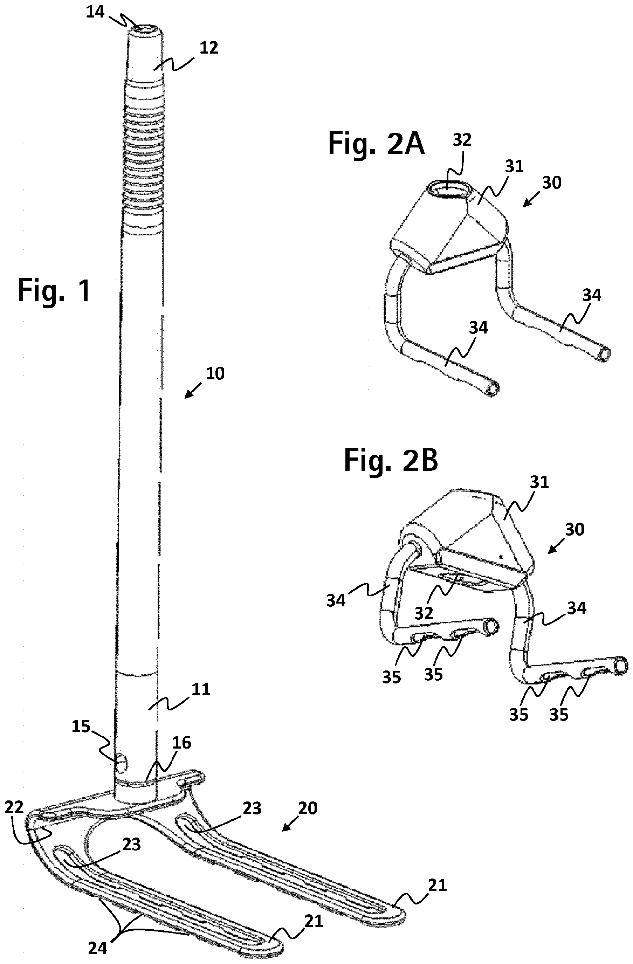

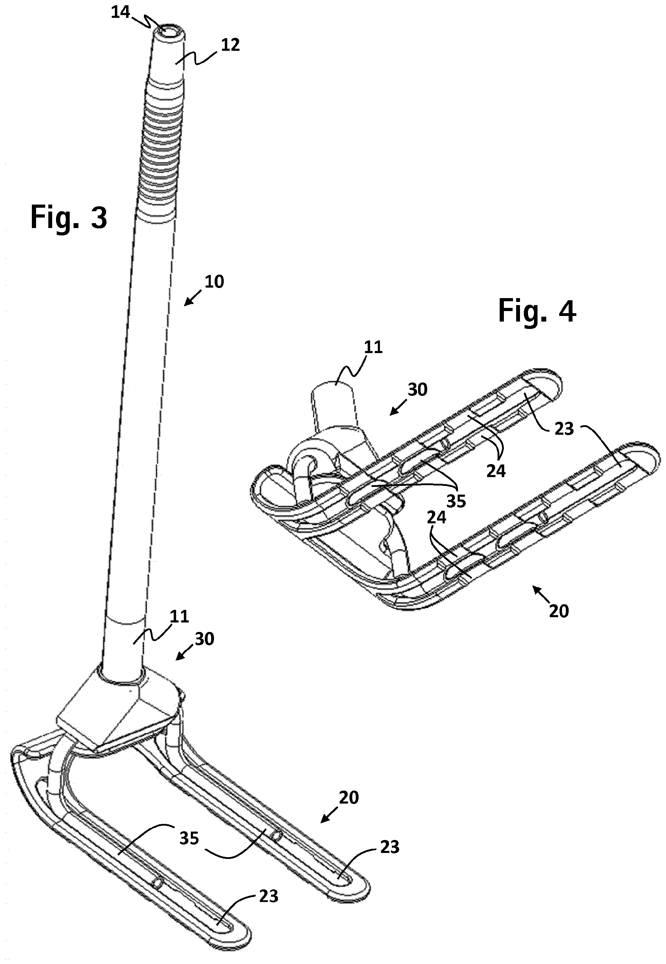

Ein erstes Ausführungsbeispiel der vorliegenden Erfindung ist im Folgenden unter Bezugnahme auf die Fig. 1 bis 6B im Detail beschrieben.

Aus der Fig. 1 geht ein Stabilisator für Operationen am Herzen hervor mit einem Befestigungsschaft 10 und einem Gewebekontaktbauteil 20 mit zwei im Wesentlichen parallelen, länglichen Auflageelementen 21, 21 welche an einem Basisabschnitt 22 vorgesehen sind und daran angepasst sind, auf zwei gegenüberliegenden Seiten eines Blutgefäßes B angeordnet zu sein, sodass zumindest ein Abschnitt des Blutgefäßes B zwischen den beiden Auflageelementen 21, 21 hindurch verläuft, wobei der Basisabschnitt 22 des Gewebekontaktbauteils 20 an einem Ende 11 des Befestigungsschafts 10 angebracht ist. Beide Auflageelemente 21, 21 weisen einen Schlitz 23 auf, der sich in Längsrichtung des Auflageelements 21 zumindest über im Wesentlichen dessen gesamte Länge erstreckt. Der Befestigungsschaft 10 weist eine axiale Bohrung 14 auf, die sich über die gesamte Länge des Befestigungsschafts 10 erstreckt und an seinem Ende 11, an welchem der Basisabschnitt 22 des Gewebekontaktbauteils 20 angebracht ist, verschlossen ist. Die axiale Bohrung 14 in dem Befestigungsschaft 10 steht mit einem Verbindungskanal 15 in Verbindung, der den axialen Kanal 14 quert. Auf diese Weise bildet der Verbindungskanal 15 an dem Befestigungsschaft 10 zwei Öffnungen in der Nähe des Endes 11 des Befestigungsschafts 10 aus.

Eine Absaugeinrichtung 30 besteht bei diesem Ausführungsbeispiel aus einem Verbindungsbauteil 31, mit dessen Hilfe die Absaugeinrichtung 30 lösbar mittels zwei 0-Ringen 40 und entsprechenden Aufnahmenuten 16 (nur eine Aufnahmenut 16 ist in den Fig. gezeigt) an dem Befestigungsschaft 10 des Stabilisators befestigbar ist, indem das Verbindungsbauteil 31 mit dessen Durchgangsbohrung 32 auf den Befestigungsschaft 10 aufgeschoben wird und dann zu dessen Ende 11 hin geschoben wird, bis die 0-Ringe 40 in den Aufnahmenuten 16 aufgenommen sind und die Bodenfläche des Verbindungsbauteils 31 an dem Befestigungsabschnitt 22 des Gewebekontaktbauteils 20 anliegt. Das Verbindungsbauteil 31 weist zudem einen Verbindungshohlraum 33 auf.

Die Absaugeinrichtung 30 besitzt zwei Absaugbauteile 34, welche aus gebogenen Rohrelementen gebildet sind. Die Absaugbauteile 34 weisen einen axialen Durchgang auf, der zu dem distalen bzw. freien Ende des Rohrelements hin offen ist. Am proximalen Ende jedes Rohrelements 34 ist es mit dem Verbindungsbauteil 31 verbunden, sodass der axiale Durchgang in jedem Rohrelement 34 mit dem Verbindungshohlraum 33 verbunden ist. Darüber hinaus weist jedes Rohrelement 34 zwei radiale Öffnungen 35 auf, die sich zu der Unterseite jedes Rohrelements 34 hin erstrecken. Die Rohrelemente 34 sind daran angepasst, teilweise in dem Schlitz 23 eines Auflageelements 21 angeordnet zu sein, wenn die Absaugeinrichtung 30 an dem Stabilisator angebracht ist. Der Außendurchmesser der Rohrelemente 34 entspricht dabei im Wesentlichen der Breite des Schlitzes 23 in dem Auflageelement 21. Die Rohrelemente 34 erstrecken sich zudem nicht über die gesamte Länge der Schlitze 23 sondern nur über etwas mehr als die halbe Länge dieser Schlitze 23.

Die Auflageelemente 21 weisen jeweils zwei Nuten 24 auf, welche sich in der Auflagefläche des Auflageelements 21 quer zu dessen Längsrichtung erstrecken. Die Nuten 24 entsprechen in Form und Größe im wesentlichen den radialen Öffnungen 35 in den Rohrelementen 34 und sind angeordnet, dass die radialen Öffnungen 35 und die Nuten 24 zumindest in manchen Bereichen des Auflageelements 21 auf einer Linie liegen, sodass Blut und andere Flüssigkeiten an der Unterseite des Auflageelements 21 entlang durch eine Nut 24 zu der radialen Öffnung 35 hin strömen kann.

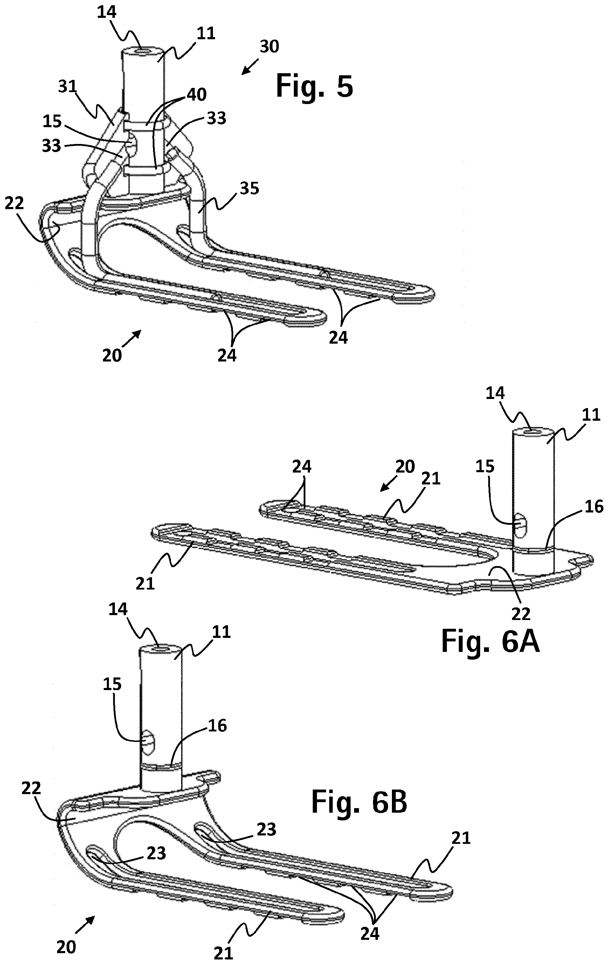

Bei dem vorliegenden Ausführungsbeispiel sind die Auflageelemente 21 nur in einer Richtung skiförmig aufgebogen, nämlich zu dem Ende hin, an dem sie mit dem Basisabschnitt 22 verbunden sind, wie dies insbesondere aus Fig. 6B ersichtlich ist. Die freien Enden der Auflageelemente 21 sind nicht aufgebogen und in Querrichtung zu den Auflageelementen 21 ist ebenfalls keine Aufbiegung vorgesehen. Allerdings sind die Kanten der Auflageelemente 21 abgerundet, sodass das Gewebe nicht durch scharfe Kanten beschädigt wird.

Der Befestigungsschaft 10 ist an seinem freien Ende 12 mit einem Anschluss versehen, nämlich einem Luer-Anschluss. An diesem Anschluss kann eine Absaugpumpe angebracht werden, wobei diese Absaugpumpe vorzugsweise speziell für chirurgische Zwecke angepasst ist. Die Absaugpumpe kann sich dabei durchgängig oder intermittierend in Betrieb befinden.

Wie dies bereits zuvor beschrieben ist, wird das Verbindungsbauteil 31 mit Hilfe von zwei 0-Ringen 40 an dem Befestigungsschaft 10 bzw. dessen Ende 11 lösbar befestigt. Die 0-Ringe 40 sind dazu in zwei umlaufenden Innennuten in dem Verbindungsbauteil 31 aufgenommen. Indem die umlaufenden Innennuten in dem Verbindungsbauteil 31 tiefer ausgebildet sind als die umlaufenden Außennuten an dem Befestigungsschaft 10, verbleiben die 0-Ringe 40 zumeist in den umlaufenden Innennuten des Verbindungsbauteils 31 und lösen sich aus den Nuten 16 in dem Befestigungsschaft. Wie dies in Fig. 5 gezeigt ist, bilden der Befestigungsschaft 11, das Verbindungsbauteil 31, die Rohrelemente 34 und die 0-Ringe 40 einen gemeinsamen Hohlraum aus, durch den Blut und anderes abgesaugt werden kann. Damit nicht zwei Hohlräume ausgebildet werden, die ausschließlich durch den Verbindungskanal 15 verbunden sind, ist in dem Verbindungsbauteil 31 noch eine weitere umlaufende Nut vorgesehen, welche die Hohlräume verbindet.

Wird nun eine Unterdruckquelle wie bspw. eine chirurgische Absaugpumpe mit einem Schlauch mit dem Luer-Anschluss des Stabilisators verbunden, kann Blut und desgleichen aus dem Wannenbereich W abgesaugt werden, ohne dass einerseits die Operation unterbrochen werden muss und andererseits das Blickfeld auf das Operationsgebiet eingeschränkt wird. Indem der Befestigungsschaft mit einem inneren Kanal ausgebildet ist, wird keine Schlauchzuleitung zu dem Gewebekontaktbauteil benötigt.

Aus den Fig. 6A und 6B ist ersichtlich, wie bei diesem Ausführungsbeispiel der Gewebekontaktabschnitt 20 erzeugt wird. Der Basisabschnitt 22 und die beiden Auflageelemente 21 sind zunächst als ebenes Bauteil ausgebildet. Die Auflageelemente 21 werden nun umgebogen, um an wenigstens einer Seite eine skiförmige Aufbiegung zu erzeugen. Eine solche skiförmige Aufbiegung ist insbesondere dahingehend vorteilhaft, dass die Belastung des darunterliegenden Gewebes im aufgebogenen Randbereich der Auflageelemente 21 verringert ist.

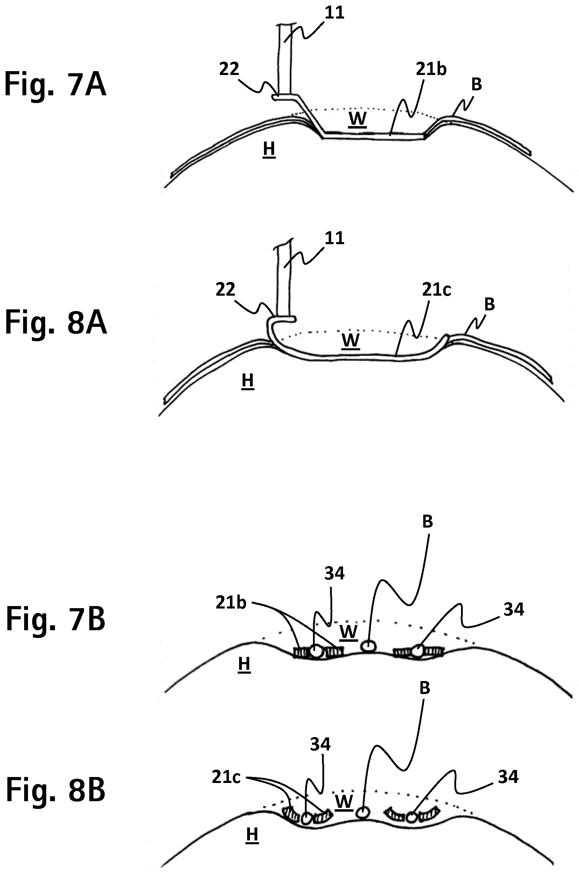

Aus den Fig. 7 und 8 wird deutlich, wie ein erfindungsgemäßer Stabilisator auf die Oberfläche des Herzens H aufgesetzt wird. Um den Stabilisator sicher zu dem Blutgefäß B zu positionieren und um eine Stabilisierung des Herzens H zu erreichen, wird der Stabilisator gegen das Herz H gedrückt. Auf diese Weise bildet sich eine wannenförmige Vertiefung W aus, die gegebenenfalls mit Blut und anderen Flüssigkeiten volllaufen kann. Die gestrichelte Linie in den Fig. 7 und 8 zeigt den Verlauf der Oberfläche des Herzend für den Fall, in dem kein Stabilisator an der Oberfläche des Herzens H positioniert wird. Insbesondere aus den Fig. 7B und 8B wird ersichtlich, dass sich die tiefsten Orte der wannenförmigen Vertiefung neben dem Blutgefäß entstehen. Genau dort sind bei dem erfindungsgemäßen Stabilisator die Absaugelemente 34 angeordnet.

Ein zweites Ausführungsbeispiel der vorliegenden Erfindung ist im Folgenden unter Bezugnahme auf die Fig. 7A und 7B im Detail beschrieben.

Wie dies aus den Fig. 7A und 7B ersichtlich ist, besitzt das Gewebekontaktbauteil 20 dieses Ausführungsbeispiels keine skiförmigen Aufbiegungen. Die beiden Auflageelemente 21b sind eben ausgebildet und über eine Abschrägung mit dem Basisabschnitt 22 verbunden. Bei einer solchen Ausbildung kann es aber an den Rändern der Auflageelemente 21b zu einer Verletzung des Herzgewebes kommen, wenn die Kanten der Auflageelemente 21 b zu scharf ausgebildet sind. Insbesondere im Randbereich der Auflageelemente 21b ist auch die Pressung des Gewebes unter den Auflageelementen 21b besonders stark, was zu Nekrosen im Gewebe führen kann.

Ein drittes Ausführungsbeispiel der vorliegenden Erfindung ist im Folgenden unter Bezugnahme auf die Fig. 8A und 8B im Detail beschrieben.

Bei diesem Ausführungsbeispiel sind die Auflageelemente 21c in allen Richtungen in der Auflageebene skiförmig aufgebogen. Auf diese Weise wird eine Gewebekontaktbauteil 20 geschaffen, welches das Herzgewebe H besonders schont.

Weitere Ausgestaltungen und Abwandlungen ergeben sich dem Fachmann aus den beigefügten Zeichnungen und den angehängten Ansprüchen. So ist es zum Beispiel möglich, die skiförmige Ausgestaltung der Auflageelemente in deren Längsrichtung entsprechend Fig. 8A mit der flächen Ausgestaltung derselben in ihrer Querrichtung entsprechend Fig. 7B zu kombinieren. Selbstverständlich können die Auflageelemente auch unterschiedlich gestaltet sein. Alle gezeigten Ausbildungen der Auflageelemente in den verschiedenen Richtungen können beliebig kombiniert werden.

## Patentansprüche

1. Stabilisator für Operationen am Herzen mit
einem Befestigungsschaft (10) und
einem Gewebekontaktbauteil (20) mit zwei im Wesentlichen parallelen, länglichen Auflageelementen (21, 21), welche an einem Basisabschnitt (22) vorgesehen sind und daran angepasst sind, auf zwei gegenüberliegenden Seiten eines Blutgefäßes (B) angeordnet zu sein, sodass zumindest ein Abschnitt des Blutgefäßes (B) zwischen den beiden Auflageelementen (21, 21) hindurch verläuft, wobei der Basisabschnitt (22) des Gewebekontaktbauteils (20) an einem Ende (11) des Befestigungsschafts (10) angebracht ist,
wobei
mindestens ein Auflageelement (21) einen Schlitz (23) aufweist, der sich in Längsrichtung des Auflageelements (21) zumindest über einen Abschnitt desselben erstreckt, wobei der Schlitz (23) das Auflageelement (21) vollständig durchdringt und daran angepasst ist, ein Absaugbauteil (34) einer Absaugeinrichtung (30) lösbar in sich anzuordnen, und
der Befestigungsschaft (10) einen axial verlaufenden Kanal (14) aufweist, welcher sich zumindest über einen Abschnitt des Befestigungsschafts (10) erstreckt und im Bereich desjenigen Endes (11) des Befestigungsschafts (10), an welchem der Basisabschnitt (22) angebracht ist, mit mindestens einem Verbindungskanal (15) in Verbindung steht, der den axial verlaufenden Kanal (14) mit der Umgebung verbindet, wobei der mindestens eine Verbindungskanal (15) radial oder schräg in dem Befestigungsschaft (10) angeordnet ist und daran angepasst ist, über ein Verbindungsbauteil (31) der Absaugeinrichtung (30) mit einem Absaugbauteil (34) der Absaugeinrichtung (30) lösbar verbunden zu werden, welches in einem Schlitz (23) des Auflageelements (21) angeordnet ist.

2. Stabilisator nach Anspruch 1, wobei
das axiale Ende (11) des Befestigungsschafts (10) verschlossen ist.

3. Stabilisator nach einem der vorangehenden Ansprüche, wobei
wenigstens ein Auflageelement (21) mindestens eine Nut (24) aufweist, welche sich in der Auflagefläche des Auflageelements (21) im Wesentlichen quer zu dessen Längsrichtung erstreckt.

4. Stabilisator nach einem der vorangehenden Ansprüche, wobei
der Befestigungsschaft (10) im Bereich des Endes (11), an dem der Basisabschnitt (22) angebracht ist, mindestens eine umlaufende Nut (16) aufweist, die in der Nähe des mindestens einen Verbindungskanals (15) angeordnet ist und daran angepasst ist, ein Dichtungselement (40) aufzunehmen.

5. Stabilisator nach einem der vorangehenden Ansprüche, wobei
die Auflagefläche der Auflageelemente (21) in Längsrichtung und/oder in Querrichtung der Auflageelemente (21) so ausgebildet ist, dass die Ränder der Auflagefläche abgerundet bzw. skispitzenartig aufgebogen sind.

6. Stabilisator nach einem der vorangehenden Ansprüche, wobei
der Befestigungsschaft (10) an seinem freien Ende (12) über einen Anschluss verfügt, an dem eine Unterdruckvorrichtung anbringbar ist, wobei der Anschluss vorzugsweise ein LUER-Anschluss ist.

7. Stabilisator nach einem der vorangehenden Ansprüche mit
einer Absaugeinrichtung (30), die lösbar an dem Befestigungsschaft (10) anbringbar ist, insbesondere durch Aufschieben auf den Befestigungsschaft (10) von dessen freiem Ende (12) her, wobei die Absaugeinrichtung (30) Folgendes aufweist:
ein Verbindungsbauteil (31), mit dessen Hilfe die Absaugeinrichtung (30) lösbar an dem Stabilisator befestigbar ist und welches wenigstens einen Verbindungshohlraum (33) aufweist,
mindestens ein Absaugbauteil (34), welches einen axial verlaufenden Kanal aufweist, der mit dem wenigstens einen Verbindungshohlraum (33) verbunden ist und welches daran angepasst ist, zumindest teilweise in einem Schlitz 23) eines Auflageelements (21) angeordnet zu sein, wenn die Absaugeinrichtung (30) an dem Stabilisator angebracht ist.

8. Stabilisator nach Anspruch 7, wobei
das mindestens eine Absaugbauteil (34) der Absaugeinrichtung (30) mindestens eine radiale Öffnung (35) aufweist, die mit einer Nut (24) in dem Auflageelement (21) in Verbindung steht, wenn die Absaugeinrichtung (30) lösbar an dem Stabilisator angebracht ist und das Absaugbauteil (34) teilweise in dem Schlitz (23) in dem Auflageelement (21) angeordnet ist.

9. Stabilisator nach einem der Ansprüche 7 oder 8, wobei
beide Auflageelemente (21, 21) je einen längs verlaufenden Schlitz (23) aufweisen, und
die Absaugeinrichtung (30) zwei Absaugbauteile (34) aufweist, welche mit dem Verbindungsbauteil (31) verbunden sind und daran angepasst sind, zumindest teilweise in dem entsprechenden Schlitz (23, 23) des Auflageelements (21, 21) angeordnet zu sein, wenn die Absaugeinrichtung (30) an dem Stabilisator angebracht ist.

10. Stabilisator nach einem der Ansprüche 7 bis 9, wobei
das Absaugbauteil (34) aus einem gebogenen Rohr besteht.

11. Stabilisator nach einem der Ansprüche 7 bis 10, wobei
das Verbindungsbauteil (31) zwei umlaufende Nuten aufweist, die an seiner axialen Durchgangsöffnung (32) derart ausgebildet sind, dass eine von diesen umlaufenden Nuten einer umlaufenden Nut (16) des Befestigungsschafts (10) gegenüber steht, wenn die Absaugeinrichtung (30) an dem Stabilisator abgebracht ist, wobei die beiden Nuten daran angepasst sind, ein Dichtungsbauteil (40), vorzugsweise einen O-Ring, aufzunehmen, wobei
das Verbindungsbauteil (31) vorzugsweise eine weitere umlaufende Nut zwischen den beiden anderen umlaufenden Nuten aufweist, welche den Verbindungshohlraum (33) zumindest teilweise bildet.

## Claims

1. Stabilizer for operations on the heart, with
a fastening shaft (10) and
a tissue contact component (20) with two substantially parallel, elongated bearing elements (21, 21), which are provided on a base portion (22) and are adapted to be arranged on two opposite sides of a blood vessel (B), such that at least a portion of the blood vessel (B) extends through between the two bearing elements (21, 21), wherein the base portion (22) of the tissue contact component (20) is attached to one end (11) of the fastening shaft (10),
wherein at least one bearing element (21) has a slit (23), which extends in the longitudinal direction of the bearing element (21) at least over a portion of the same, wherein the slit (23) passes right through the bearing element (21) and is adapted to have a suction component (34) of a suction device (30) releasably arranged in it, and
the fastening shaft (10) has an axially extending channel (14), which extends at least over a portion of the fastening shaft (10) and, in the area of that end (11) of the fastening shaft (10) to which the base portion (22) is attached, is in connection with at least one connection channel (15) that connects the axially extending channel (14) to the surroundings,
wherein the at least one connection channel (15) is arranged radially or obliquely in the fastening shaft (10) and is adapted to be releasably connected by way of a connection component (31) of the suction device (30) to a suction component (34) of the suction device (30), which is arranged in a slit (23) of the bearing element (21) .

2. Stabilizer according to Claim 1, wherein
the axial end (11) of the fastening shaft (10) is closed.

3. Stabilizer according to one of the preceding claims,
wherein
at least one bearing element (21) has at least one groove (24), which extends in the bearing surface of the bearing element (21) substantially transversely in relation to the longitudinal direction thereof.

4. Stabilizer according to one of the preceding claims,
wherein
the fastening shaft (10) has in the area of the end (11) to which the base portion (22) is attached at least one peripheral groove (16), which is arranged in the vicinity of the at least one connection channel (15) and is adapted to receive a sealing element (40).

5. Stabilizer according to one of the preceding claims,
wherein
the bearing surface of the bearing elements (21) is formed in the longitudinal direction and/or in the transverse direction of the bearing elements (21) such that the edges of the bearing surface are rounded off or bent up in the manner of the tips of skis.

6. Stabilizer according to one of the preceding claims,
wherein
the fastening shaft (10) has at its free end (12) a connection to which a vacuum device can be attached, wherein the connection is preferably a LUER connection.

7. Stabilizer according to one of the preceding claims
with
a suction device (30), which can be releasably attached to the fastening shaft (10), in particular by being pushed onto the fastening shaft (10) from the free end (12) thereof, wherein the suction device (30) has the following:
a connection component (31), with the aid of which the suction device (30) can be releasably fastened to the stabilizer and which has at least one connection cavity (33),
at least one suction component (34), which has an axially extending channel, which is connected to the at least one connection cavity (33) and is adapted to be arranged at least partially in a slit (23) of a bearing element (21) when the suction device (30) is attached to the stabilizer.

8. Stabilizer according to Claim 7, wherein
the at least one suction component (34) of the suction device (30) has at least one radial opening (35), which is in connection with a groove (24) in the bearing element (21) when the suction device (30) is releasably attached to the stabilizer and the suction component (34) is arranged partially in the slit (23) in the bearing element (21).

9. Stabilizer according to either of Claims 7 and 8,
wherein
both bearing elements (21, 21) each have a longitudinally extending slit (23), and
the suction device (30) has two suction components (34), which are connected to the connection component (31) and are adapted to be arranged at least partially in the corresponding slit (23, 23) of the bearing element (21, 21) when the suction device (30) is attached to the stabilizer.

10. Stabilizer according to one of Claims 7 to 9, wherein the suction component (34) consists of a bent tube.

11. Stabilizer according to one of Claims 7 to 10, wherein
the connection component (31) has two peripheral grooves, which are formed at its axial through-opening (32) in such a way that one of these peripheral grooves is opposite a peripheral groove (16) of the fastening shaft (10) when the suction device (30) is attached to the stabilizer,
wherein the two grooves are adapted to receive a sealing component (40), preferably an O-ring, wherein
the connection component (31) preferably has between the two other peripheral grooves a further peripheral groove, which at least partially forms the connection cavity (33).

## Revendications

1. Stabilisateur pour opérations cardiaques, comprenant
une tige de fixation (10) et
un composant de contact avec les tissus (20), comprenant deux éléments d'appui allongés, essentiellement parallèles (21, 21), qui sont prévus au niveau d'une portion de base (22) et qui sont prévus pour être disposés sur deux côtés opposés d'un vaisseau sanguin (B), de telle sorte qu'au moins une portion du vaisseau sanguin (B) s'étende entre et à travers les deux éléments d'appui (21, 21), la portion de base (22) du composant de contact avec les tissus (20) étant montée à une extrémité (11) de la tige de fixation (10),
au moins un élément d'appui (21) présentant une fente (23) qui s'étend dans la direction longitudinale de l'élément d'appui (21) au moins sur une portion de celui-ci, la fente (23) traversant complètement l'élément d'appui (21) et étant prévue pour recevoir de manière amovible un composant d'aspiration (34) d'un dispositif d'aspiration (30), et
la tige de fixation (10) présentant un canal s'étendant axialement (14), lequel s'étend au moins sur une portion de la tige de fixation (10) et, dans la région de l'extrémité (11) de la tige de fixation (10) au niveau de laquelle la portion de base (22) est montée, étant en liaison avec au moins un canal de liaison (15) qui relie le canal s'étendant axialement (14) à l'environnement, l'au moins un canal de liaison (15) étant disposé radialement ou obliquement dans la tige de fixation (10) et étant prévu pour être relié de manière amovible par le biais d'un composant de liaison (31) du dispositif d'aspiration (30) à un composant d'aspiration (34) du dispositif d'aspiration (30), lequel composant d'aspiration est disposé dans une fente (23) de l'élément d'appui (21).

2. Stabilisateur selon la revendication 1, dans lequel l'extrémité axiale (11) de la tige de fixation (10) est fermée.

3. Stabilisateur selon l'une quelconque des revendications précédentes, dans lequel
au moins un élément d'appui (21) présente au moins une rainure (24) qui s'étend dans la surface d'appui de l'élément d'appui (21) essentiellement transversalement à sa direction longitudinale.

4. Stabilisateur selon l'une quelconque des revendications précédentes, dans lequel
la tige de fixation (10), dans la région de l'extrémité (11) au niveau de laquelle est montée la portion de base (22), présente au moins une rainure périphérique (16) qui est disposée à proximité de l'au moins un canal de liaison (15) et qui est prévue pour recevoir un élément d'étanchéité (40) .

5. Stabilisateur selon l'une quelconque des revendications précédentes, dans lequel
la surface d'appui des éléments d'appui (21) dans la direction longitudinale et/dans la direction transversale des éléments d'appui (21) est réalisée de telle sorte que les bords de la surface d'appui soient arrondis ou soient recourbés en forme de pointe de ski.

6. Stabilisateur selon l'une quelconque des revendications précédentes, dans lequel
la tige de fixation (10) dispose, au niveau de son extrémité libre (12), d'un raccord au niveau duquel un dispositif à dépression peut être monté, le raccord étant de préférence un raccord de LUER.

7. Stabilisateur selon l'une quelconque des revendications précédentes, comprenant
un dispositif d'aspiration (30) qui peut être monté de manière amovible sur la tige de fixation (10), en particulier en le poussant sur la tige de fixation (10) à partir de son extrémité libre (12), le dispositif d'aspiration (30) présentant les éléments suivants :
un composant de liaison (31) à l'aide duquel le dispositif d'aspiration (30) peut être fixé de manière amovible au stabilisateur, et qui présente au moins une cavité de liaison (33),
au moins un composant d'aspiration (34) qui présente un canal s'étendant axialement, qui est relié à l'au moins une cavité de liaison (33) et qui est prévu pour être disposé au moins en partie dans une fente (23) d'un élément d'appui (21) lorsque le dispositif d'aspiration (30) est monté sur le stabilisateur.

8. Stabilisateur selon la revendication 7, dans lequel l'au moins un composant d'aspiration (34) du dispositif d'aspiration (30) présente au moins une ouverture radiale (35) qui est en liaison avec une rainure (24) dans l'élément d'appui (21) lorsque le dispositif d'aspiration (30) est monté de manière amovible sur le stabilisateur, et le composant d'aspiration (34) est disposé en partie dans la fente (23) dans l'élément d'appui (21).

9. Stabilisateur selon l'une quelconque des revendications 7 ou 8, dans lequel les deux éléments d'appui (21, 21) présentent chacun une fente s'étendant longitudinalement (23), et
le dispositif d'aspiration (30) présente deux composants d'aspiration (34) qui sont reliés au composant de liaison (31) et qui sont prévus pour être disposés au moins en partie dans la fente correspondante (23, 23) de l'élément d'appui (21, 21) lorsque le dispositif d'aspiration (30) est monté sur le stabilisateur.

10. Stabilisateur selon l'une quelconque des revendications 7 à 9, dans lequel
le composant d'aspiration (34) se compose d'un tuyau coudé.

11. Stabilisateur selon l'une quelconque des revendications 7 à 10, dans lequel
le composant de liaison (31) comprend deux rainures périphériques qui sont réalisées au niveau de son ouverture de passage axiale (32) de telle sorte que l'une de ces rainures périphériques soit opposée à une rainure périphérique (16) de la tige de fixation (10) lorsque le dispositif d'aspiration (30) est monté sur le stabilisateur, les deux rainures étant prévues pour recevoir un composant d'étanchéité (40), de préférence un joint torique,
le composant de liaison (31) présentant de préférence une rainure périphérique supplémentaire entre les deux autres rainures périphériques, laquelle forme au moins en partie la cavité de liaison (33).
